(19) Europäisches Patentamt
European Patent Office
Office européen des brevets

(11) Veröffentlichungsnummer: **0 239 035**
**B1**

(12) # EUROPÄISCHE PATENTSCHRIFT

(45) Veröffentlichungstag der Patentschrift: **04.07.90**

(51) Int. Cl.⁵: **C08F 220/04**, C08F 220/54, C08F 220/34

(21) Anmeldenummer: **87104143.0**

(22) Anmeldetag: **20.03.87**

(54) **Verfahren zur Herstellung von feinteiligen pulverförmigen vernetzten Copolymerisaten und deren Verwendung.**

(30) Priorität: **22.03.86 DE 3609829**

(43) Veröffentlichungstag der Anmeldung: **30.09.87 Patentblatt 87/40**

(45) Bekanntmachung des Hinweises auf die Patenterteilung: **04.07.90 Patentblatt 90/27**

(84) Benannte Vertragsstaaten: **AT BE CH DE ES FR GB IT LI NL SE**

(56) Entgegenhaltungen:
**EP-A- 0 220 603**
**GB-A- 1 172 713**

(73) Patentinhaber: **BASF Aktiengesellschaft, Carl-Bosch-Strasse 38, D-6700 Ludwigshafen(DE)**

(72) Erfinder: **Hartmann, Heinrich, Dr., Weinheimer Strasse 46, D-6703 Limburgerhof(DE)**
Erfinder: **Denzinger, Walter, Wormser Landstrasse 65, D-6720 Speyer(DE)**

EP 0 239 035 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Pulverförmige vernetzte Copolymerisate können nach verschiedenen Verfahren durch Copolymerisieren der Monomeren in Gegenwart eines Vernetzers hergestellt werden, z.B. nach dem Verfahren der Fällungspolymerisation in Benzol oder chlorierten Kohlenwasserstoffen gemäß der US-PS 2 798 053 oder der umgekehrten Suspensionspolymerisation nach dem Verfahren der US-PS 4 059 552. In allen Fällen benötigt man ein Verdünnungsmittel. Bei der Herstellung von Verdickungsmitteln auf Basis vernetzter Polyacrylsäuren bzw. Polyacrylamide haben sich vor allem Benzol, Toluol, Xylol oder halogenierte Kohlenwasserstoffe als inertes Verdünnungsmittel bewährt. Der Einsatz dieser Verdünnungsmittel ist jedoch auch mit einem Nachteil behaftet, weil die darin hergestellten Copolymerisate auch nach dem Trocknen noch mehr oder weniger größere Mengen an inertem Verdünnungsmittel, wie aromatischen Kohlenwasserstoffen oder halogenierten Kohlenwasserstoffen enthalten. Da die genannten Kohlenwasserstoffe zum Teil als physiologisch bedenklich angesehen werden bzw. den Copolymerisation einen intensiven Geruch verleihen, müssen sie weitgehend aus den Copolymerisaten entfernt werden. Dadurch werden Reinigungsoperationen erforderlich, die umständlich und kostenaufwendig sind und darüber hinaus nicht die vollständige Entfernung der störenden Lösemittel gestatten.

Aus der US-PS 3 522 228 ist ein Verfahren zur Herstellung von Polymerisation bekannt, bei dem man ethylenisch ungesättigte Monomere in Gegenwart von Katalysatoren in flüssigem Kohlendioxid bei Temperaturen von –78 bis 100°C unter erhöhtem Druck der Homo- oder Copolymerisation unterwirft. Die Polymerisate fallen in Form von gröberen Pulvern oder viskosen Ölen an, aus denen sie durch Behandlung mit Flüssigkeiten, in denen sich die Polymerisate nicht lösen, ausgefällt werden.

Der vorliegenden Erfindung liegt die Aufgabe zugrunde, ein Verfahren zur Verfügung zu stellen, gemäß dem pulverförmige vernetzte Copolymerisate durch Copolymerisation der Monomeren in einem Verdünnungsmittel erhalten werden, das physiologisch unbedenklich ist und aus den Polymerisaten leicht entfernt werden kann.

Die Aufgabe wird erfindungsgemäß mit einem Verfahren zur Herstellung von pulverförmigen vernetzten Copolymerisation durch Polymerisieren von Monomerenmischungen aus

(a) 70 bis 99,99 Gew.% monoethylenisch ungesättigten Carbonsäuren, Amiden von monoethylenisch ungesättigten Carbonsäuren und/oder Estern aus monoethylenisch ungesättigten Carbonsäuren und Aminoalkoholen der Formel

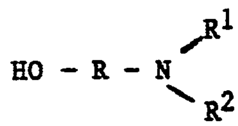 ,

in der R = $C_2$- bis $C_6$-Alkylen,
$R^1$, $R^2$ = H, $CH_3$, $C_2H_5$, $C_3H_7$ bedeutet,

(b) 0,001 bis 10 Gew.% eines mindestens zweifach ethylenisch ungesättigten Monomeren und

(c) 0 bis 20 Gew.% anderen monoethylenisch ungesättigten Monomeren

in einem inerten Verdünnungsmittel in Gegenwart von Radikale bildenden Initiatoren und Entfernung des Verdünnungsmittels, wenn man die Copolymerisation unter Druck in überkritischem Kohlendioxid als inertem Verdünnungsmittel unter Durchmischung bei Temperaturen bis 150°C und Drücken oberhalb von 73 bar durchführt und auf 100 Gew.-Teile der Monomeren 100 bis 1500 Gew.-Teile Kohlendioxid einsetzt.

Als Monomere der Gruppe (a) kommen zunächst monoethylenisch ungesättigte Carbonsäuren in Betracht. Die wichtigsten Vertreter dieser Gruppe sind beispielsweise monoethylenisch ungesättigte $C_3$- bis $C_5$-Carbonsäuren, wie Acrylsäure, Methacrylsäure, Vinylessigsäure, Itaconsäure, Crotonsäure, alpha-Methylcrotonsäure, alpha-Ethylacrylsäure, Dimethylacrylsäure, alpha-Chloracrylsäure und Vinylmilchsäure. Ebenfalls geeignet sind die Amide von monoethylenisch ungesättigten Carbonsäuren, z.B. Acrylamid, Methacrylamid, Crotonsäureamid und Itaconsäureamid. Zu den Monomeren der Gruppe (a) gehören außerdem Ester aus monoethylenisch ungesättigten Carbonsäuren und Aminoalkoholen der Formel

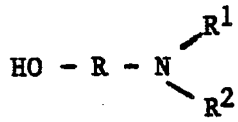 ,

in der R = $C_2$- bis $C_6$-Alkylen,
$R^1$, $R^2$ = H, $CH_3$, $C_2H_5$, $C_3H_7$ bedeutet. Vorzugsweise handelt es sich hierbei um Di-$C_1$- bis $C_3$-Alkylamino-$C_2$- bis $C_6$-alkylacrylate bzw. -methacrylate. Einzelne Vertreter dieser Monomeren sind beispielsweise Dimethylaminoethylacrylat, Dimethylaminoethylmethacrylat, Diethylaminoethylmethacrylat, Diethylaminoethylacrylat, Dipropylaminoethylacrylat, Dimethylaminopropylacrylat, Dimethylaminopropylmethacrylat, Dimethylaminobutylacrylat, Dimethylaminobutylmethacrylat, Dimethylaminoneopentylacrylat, Dimethylaminoneopentylmethacrylat und Dimethylaminohexylacrylat. Die genannten basischen Acrylate und Methacrylate werden vorzugsweise in Form der Salze mit Mineralsäuren oder Carbonsäuren (z.B. Salzsäure, Schwefelsäure, Ameisensäure, Essigsäure oder Propionsäure) oder in quaternisierter Form eingesetzt. Geeignete Quaternisierungsmittel sind beispielsweise Dimethylsulfat, Methylchlorid, Ethylchlorid, Laurylchlorid oder Benzylchlorid.

Die Monomeren der Gruppe (a) können entweder für sich allein oder in Mischung der Copolymerisation mit den Monomeren (b) unterworfen werden. Von den Monomeren der Gruppe (a) verwendet man vorzugsweise Acrylsäure, Methacrylsäure, Acrylamid und/oder Methacrylamid. Verwendet man als Monomer (a) Acrylsäure, so erhält man durch Copolymerisation mit den Monomeren der Gruppe (b) eine vernetzte Polyacrylsäure, entsprechend liefert der Einsatz von Acrylamid als Monomer der Gruppe (a) vernetzte Polyacrylamide. Setzt man als Monomere der Gruppe (a) Mischungen aus Acrylsäure und Acrylamid ein, so erhält man bei der Copolymerisation vernetzte Copolymerisate aus Acrylamid und Acrylsäure. Eine weitere Variation der vernetzten Copolymerisate wird dadurch möglich, daß man entweder Acrylsäure zusätzlich in Gegenwart von Dimethylaminoethylacrylat oder Acrylamid in Gegenwart von Dimethylaminoethylacrylat zusammen mit den Monomeren (b) und gegebenenfalls den Monomeren der Gruppe (c) der Copolymerisation unterwirft. Da die Monomeren der Gruppe (a) in jedem beliebigen Verhältnis miteinander copolymerisierbar sind, ergibt sich eine Vielfalt von Variationsmöglichkeiten bezüglich der Zusammensetzung der vernetzten Copolymerisate. Die Monomeren der Gruppe (a) sind zu 70 bis 99,95 Gew.% am Aufbau der Copolymerisate beteiligt.

Als Monomere der Gruppe (b) verwendet man mindestens zweifach ethylenisch ungesättigte Doppelbindungen enthaltende Monomere, z.B. Divinylbenzol, Divinylketon, Butandioldiacrylat, Ethylenglykoldiacrylat, Butandioldimethacrylat, Ethylenglykoldimethacrylat, Methylenbisacrylamid, Diallylphthalat, Divinylether, Divinyldioxan, Polyalkenylpolyether wie Polyallyl- und Polyvinylether von Mono-, Di- und Oligosacchariden, z.B. Tri-, Tetra-, Penta- und Hexaallylsaccharose sowie deren Gemische, Pentaerythrittriallylether, Di- und Triallylether von Trimethylolpropan und Diallylacrylamid, Polyallyl- und Polyvinylsilane, Triallylcyanurat, Allylester der Phosphorsäure und phosphorigen Säure sowie Ally phosphoramidoverbindungen, wie Phosphorsäuremonoethylester-N,N-(diallyl)- -diamid sowie Mischungen dieser Monomeren. Vorzugsweise verwendet man als Vernetzter Divinyldioxan, Di-, Tri-, Tetra- und Penta-allylsaccharose, Pentaerythrittriallylether und/oder N,N'-Methylen-bisacrylamid. Die Vernetzer können entweder allein oder in Mischung bei der Copolymerisation angewendet werden. Die Monomeren der Gruppe (b) sind in den bei der Polymerisation eingesetzten Monomerenmischungen in einer Menge von 0,001 bis 10, vorzugsweise 0,05 bis 5 Gew.% answesend.

Zur Modifizierung der vernetzten Copolymerisate kann man bei der Copolymerisation andere ethylenisch ungesättigte Monomere einsetzen. Zu der Gruppe von Monomeren (c) gehören beispielsweise Acrylnitril, Methacrylnitril, Acrylsäure- und Methacrylsäureester, die sich von einwertigen $C_1$- bis $C_{18}$-Alkoholen ableiten, Hydroxy-$C_2$- bis $C_4$-Alkylester der Acrylsäure und Methacrylsäure, Maleinsäureanhydrid, Vinylester, 2-Acrylamido-2-methylpropylsulfonsäure und/oder Vinylphosphonsäure. Außerdem eignen sich Ester der Acrylsäure und Methacrylsäure mit Fettalkoholethoxylaten und Fettalkoholpropoxylaten, wobei die Fettalkoholkomponente 10 bis 20 C-Atome besitzt und der Ethylenoxid- bzw. Propylenoxidanteil 1 bis 20 Mol% beträgt. Solche Alkoholkomponenten werden beispielsweise dadurch erhalten, daß man $C_{10}$- bis $C_{20}$-Fettalkohole mit Ethylenoxid und/oder Propylenoxid umsetzt und die dabei erhaltenen alkoxylierten Fettalkohole mit Acrylsäure bzw. Methacrylsäure verestert. Der Einsatz dieser Comonomeren ergibt vernetzte Copolymerisate, die eine hohe Elektrolytbeständigkeit aufweisen. Die Monomeren der Gruppe (c) werden in einer Menge von 0 bis 20, und vorzugsweise bis 15 Gew.% eingesetzt. Sofern sie für die Modifizierung der Copolymerisate aus (a) und (b) verwendet werden, beträgt die untere Grenze 5 Gew.%, bezogen auf die Monomerenmischung. Die Summe der Prozentangaben für die Monomeren (a), (b) und (c) beträgt in allen Fällen 100 %. Ester der Acrylsäure und Methacrylsäure sind beispeilsweise Methylacrylat, Ethylacrylat, Methylmethacrylat, 2-Ethylhexylacrylat, Stearylacrylat, Stearylmethacrylat und die Acrylsäureester der isomeren Butylalkohole. Als Hydroxy-$C_2$-bis-$C_4$-alkylester der Acrylsäure und Methacrylsäure kommen beispielsweise Hydroxy ethylacrylate, Hydroxypropylacrylat, Hydroxybutylacrylat, Hydroxyethylmethacrylat, Hydroxypropylmethacrylat sowie Hydroxybutylmethacrylat in Betracht. Von den Vinylestern werden vorzugsweise Vinylacetat und Vinylpropionat eingesetzt.

Die Copolymerisation wird unter Druck in überkritischem Kohlendioxid als inertem Verdünnungsmittel durchgeführt. Über die Eigenschaften von Kohlendioxid in flüssigem und im überkritischen Zustand berichtete <u>J.A. Hyatt</u>, J. Org. Chem. <u>49</u>, 5097-5101 (1984). Danach liegt der kritische Punkt von Kohlendioxid bei etwa 31°C und 73 bar. Die Copolymerisation wird in allen Fällen unter Druck in überkritischem Kohlendioxid bei Temperaturen von oberhalb etwa 31°C, der kritischen Temperatur des Kohlendioxids, vorgenommen. Als obere Grenze für die Herstellung der Copolymerisate wird diejenige Temperatur angesehen, die 10°C unterhalb des beginnenden Erweichungsbereiches der jeweils entstehenden Copolymerisate liegt. Der obere Wert für diese Temperaturgrenze beträgt für die meisten Copolymerisate 150°C. Die Copolymerisation wird vorzugsweise in dem Temperaturbereich von 40 bis 130°C durchgeführt. Die Drücke liegen dabei oberhalb von 73 bar, vorzugsweise in dem Bereich von 80 bis 300 bar.

Die Polymerisationsreaktion wird mit Hilfe von in Radikale zerfallende Polymerisationsinitiatoren gestartet. Es können sämtlich Initiatoren eingesetzt werden, die für die Polymerisation der Monomeren bekannt sind. Geeignet sind beispielsweise in Radikale zerfallende Initiatoren, die bei den jeweils gewählten Temperaturen eine Halbwertszeit von weniger als 3 Stunden besitzen. Falls die Polymerisation bei zwei unterschiedlichen Temperaturen durchgeführt wird, indem man die Monomeren zunächst bei einer niedrigeren Temperatur anpolymerisiert und anschließend bei einer deutlich höheren Temperatur auspolymerisiert, so verwendet man zweckmäßigerweise mindestens zwei unterschiedliche Initiatoren, die in dem jeweils gewählten Temperaturbereich eine ausreichende Zerfallsgeschwindigkeit haben. Beispielsweise kann man in den nachstehend angegebenen Temperaturbereichen folgende Initiatoren verwenden:

Temp: 40 bis 60°C

Acetylcyclohexansulfonylperoxid, Diacetylperoxidicarbonat, Dicyclohexylperoxidicarbonat, Di-2-ethylhexylperoxidicarbonat, tert.-Butylperneodecanoat, 2,2'-Azobis-(4-methoxi-2,4-dimethylvaleronitril)

Temp.: 60 bis 80°C:

tert.-Butylperpivalat, Dioctanoylperoxid, Dilauroylperoxid, 2,2'-Azobis-(2,4-dimethylvaleronitril), tert.-Butylazo-2-cyanobutan

Temp.: 80 bis 100°C:

Dibenzoylperoxid, tert.-Butylper-2-ethylhexanoat, tert.-Butylpermaleinat, 2,2-Azobis-(isobutyronitril)

Temp.: 100 bis 120°C:

Bis-(tert.-butylperoxi)-cyclohexan, tert.-Butylperoxiisopropylcarbonat, tert.-Butylperacetat

Temp.: 120 bis 140°C:

2,2-Bis-(tert.-butylperoxi)-butan, Dicumylperoxid, Di-tert.-amylperoxid, Di-tert.-butylperoxid

Temp.: 140°C bis 160°C:

p-Methanhydroperoxid, Pinanhydroperoxid, Cumolhydroperxoxid, tert.-Butylhydroperoxid.

Durch die Mitverwendung von Redox-Coinitiatoren, beispielsweise Benzoin, Dimethylanilin sowie organisch löslicher Komplexe und Salze von Schwermetallen, wie Kupfer, Kobalt, Mangan, Eisen, Nickel und Chrom, können die Halbwertszeiten der genannten Peroxide, besonders der Hydroperoxide, verringert werden, so daß beispielsweise tert.-Butylhydroperoxid in Gegenwart von 5 ppm Kupfer-II-Acetylacetonat bereits bei 100°C wirksam ist.

Die Polymerisationsinitiatoren werden in den bei Polymerisationen üblichen Mengen eingesetzt, z.B. benötigt man pro 100 Gew.Teile der Monomeren 0,05 bis 10, vorzugsweise 0,1 bis 5 Gew.Teile eines Initiators.

Die Polymerisation kann gegebenenfalls auch in Gegenwart von Polymerisationsreglern durchgeführt werden, um das Molekulargewicht der Polymerisate zu regeln. Sofern man besonders niedrigmolekulare Copolymerisate herstellen will, setzt man höhere Mengen an Polymerisationsreglern ein, während man für die Herstellung von hochmolekularen Copolymerisation nur geringe Mengen an Polymerisationsreglern verwendet bzw. in Abwesenheit dieser Stoffe arbeitet. Geeignete Polymerisationsregler sind beispielsweise 2-Mercapto-ethanol, Mercaptopropanole, Mercaptobutanole, Thioglykolsäure, N-Dodecylmercaptan, tert.-Dodecylmercaptan, Thiophenol, Mercaptopropionsäure, Allylalkohol und Acetaldehyd. Die Polymerisationsregler werden, bezogen auf die eingesetzten Monomeren, in einer Menge von 0 bis 10, vorzugsweise 0,1 bis 5 Gew.%, eingesetzt.

Bezogen auf 100 Gew.Teile der Monomerenmischung verwendet man 100 bis 1500, vorzugsweise 200 bis 900 Gew.Teile Kohlendioxid. Es ist vorzugsweise wasserfrei. Die Polymerisationsreaktion kann diskontinuierlich oder auch kontinuierlich unter Durchmischung der Reaktionspartner in entsprechend ausgelegten Druckapparaturen durchgeführt werden. Um die bei der Polymerisation entstehende Wärme abzuführen, ist es wünschenswert, daß die Druckapparaturen über ein Kühlsystem verfügen. Sie müssen selbstverständlich ebenso auch beheizbar sein, um die Reaktionsmischung auf die jeweils für die Polymerisation gewünschte Temperatur zu erhitzen. Die Druckapparaturen sollten über Mischeinrichtungen verfügen, z.B. Rührer (Blatt-, Impeller-, Mehrstufenimpulsgegenstrom-Rührer) oder Schaufeln.

Die Druckpolymerisation kann beispielsweise so asugeführt werden, daß man in einer Druckapparatur zunächst die Monomeren und den Initiator vorlegt, dann Kohlendioxid in flüssiger Form einbringt und

nach dem Verschließen des Autoklaven das Reaktionsgemisch auf die Polymerisationstempteratur erhitzt. Es ist jedoch auch möglich, nur einen Teil des Reaktionsgemisches im Autoklaven vorzulegen und es auf die Polymerisationstempteratur zu erhitzen und dann nach Maßgabe der Polymerisation weiteres Reaktionsgemisch zuzupumpen. Eine andere Möglichkeit besteht darin, nur einen Teil der Monomeren in der gesamten erforderlichen Menge an Kohlendioxid im Autoklaven vorzulegen und die Monomeren zusammen mit dem Initiator nach Fortschritt der Polymerisation in den Autoklaven einzupumpen. Nach Abschluß der Polymerisationsreaktion wird das Reaktionsgemisch gegebenenfalls gekühlt und das Kohlendioxid abgetrennt. Dabei fallen die Copolymerisate als pulverförmiger Rückstand an. Die Teilchengröße des Pulvers beträgt 0,5 µm bis 5 mm, vorzugsweise 1 µm bis 0,5 mm, wobei diese Teilchen aus Primärteilchen von 0,5 bis 3 µm Durchmesser bestehen und mehr oder weniger locker aggregiert sind. Das abgetrennte Kohlendioxid kann wieder verflüssigt und erneut – ohne aufwendige Reinigung – bei der Polymerisation wiederverwendet werden.

Die gemäß Erfindung hergestellten hochmolekularen vernetzten Copolymerisate können beispielsweise als Verdickungsmittel für wäßrige Systeme verwendet werden, z.B. in Textildruckpasten, kosmetischen und pharmazeutischen Zubereitungen, Dispersionsfarben, Klebstoffen und Erdölbohrspülmitteln. Die hochmolekularen vernetzten Copolymerisate auf Basis von Acrylsäure und/oder Methacrylsäure werden vorzugsweise in Mengen von 0,1 bis 5 Gew.-% in kosmetischen und pharmazeutischen Zubereitungen, wie Cremes und Salben, verwendet.

Diejenigen vernetzten Copolymerisate, die freie Carboxylgruppen enthalten, ergeben nach Neutralisation mit einer wäßrigen Base klare Gele, die je nach Anwendungskonzentration als fließfähig bis steif zu beurteilen sind. Die Verdickungswirkung wird bei diesen Copolymerisation durch Neutralisation der Carboxylgruppen erreicht, wobei die optimale Verdickungswirkung in dem pH-Bereich von 6,0 bis 10,0 vorzugsweise 7,0 bis 9,0, eintritt. Zur Neutralisation verwendet man hauptsächlich Natriumhydroxid, Kaliumhydroxid, Natrium- oder Kaliumcarbonat, Ammoniak oder in Mischungen dieser Basen sowie Amine, z.B. Tri-$C_1$-bis $C_5$-alkylamine oder Mono-, Di- oder Tri-$C_1$-bis $C_4$-alkanolamine oder deren Mischungen. Vernetzte Copolymerisate auf Basis von Amiden ergeben in wäßrigen Systemen sowohl im sauren als auch im alkalischen pH-Bereich Viskositätserhöhungen. Vernetzte Dialkylaminoalkylacrylate und -methacrylate führen im sauren pH-Bereich zu einer starken Viskositätszunahme des wäßrigen Systems. Um bei der Prüfung der Verdickerwirkung der vernetzten Copolymerisate reproduzierbare Werte zu erhalten, wurde die Viskosität des verdickten wäßrigen Systems jeweils folgendermaßen gemessen:

In einem 600 ml fassenden Becherglas wurden 490 g vollentsalztes Wasser vorgelegt. Unter Rühren mit einem Flügelrührer bei einer Drehzahl von ca. 100 Umdrehungen/min streute man dann 5 g des pulverförmigen vernetzten Copolymerisates so ein, daß eine Verklumpung des Pulvers im Wasser vermieden wurde. Die Mischung wurde 10 Minuten gerührt, dann 10 Minuten stehen gelassen, anschließend mit 5 g wäßrigem Ammoniak (22 %ig) versetzt und danach homogenisiert. Der pH-Wert des Ansatzes soll 8 bis 9 betragen. Um praxisnahe Ergebnisse zu erhalten, wird eine Probe von 250 g des Ansatzes mit einem Schnellrührer (Propellerdurchmesser 3,5 cm) bei 8000 Umdrehungen/min 3 Minuten gerührt. Die Temperatur dieser Probe wird anschließend auf 20°C eingestellt. Nach dem Temperieren der Probe wird die Viskosität in einem Haake-Viskotester VT 24 bei 5,66 Umdrehungen/min gemessen.

Die in den Beispielen angegebenen Teile sind Gewichtsteile, die Angaben in Prozent beziehen sich auf das Gewicht der Stoffe.

Beispiel 1

In einem 300 ml fassenden Autoklaven werden 15 g Acrylsäure, 0,075 g Pentaerythrittriallylether und 0,075 g tert.-Butylperethylhexanoat vorgelegt. Man fügt 135 g flüssiges Kohlendioxid zu und verschließt den Autoklaven, der mit einem Rührer und einer elektrischen Heizung ausgestattet ist. Das Reaktionsgemisch wird mit 300 Umdrehungen/min gerührt und auf eine Temperatur von 80°C erwärmt. Dabei stellt sich ein maximaler Druck von 150 bar ein. Nach einer Verweilzeit des Reaktionsgemisches von 5 Stunden bei einer Temperatur von 80°C und 150 bar wird der Autoklaveninhalt gekühlt und entspannt. Man erhält ein weißes lockeres Pulver, das sich aus Aggregaten einer Teilchengröße von 15 bis 500 µm zusammensetzt. Die Größe der Primärteilchen beträgt ca. 1 µm. Nach der oben beschriebenen Prüfmethode für die Verdickungswirkung von Copolymerisaten wird eine Viskosität der wäßrigen Lösung von 7.000 mPas erhalten.

Beispiele 2 bis 16

Beispiel 1 wird mit den der folgenden Tabelle angegebenen Einsatzstoffen sowie unter den übrigen Bedingungen (Temperatur, Druck und Zeit) wiederholt. In allen Fällen erhält man ein weißes lockeres pulverförmiges Copolymerisat, das eine Teilchengröße von 15 bis 500 µm hat. Die Teilchengröße der Primärteilchen liegt jeweils in dem Bereich von ca. 1 bis 3 µm. Die Copolymerisate sind Verdickungsmittel, die Verdickungswirkung gemäß der oben angegebenen Prüfvorschrift ist ebenfalls in der Tabelle angegeben.

Tabelle

| Beispiel | $CO_2$ [g] | Monomer (a) [g] | 0 ggf. (c) | Monomer (b) [g] | | Initiator [g] | Temp. [°C] | Druck [bar] | Zeit [h] | Verdickungswirkung (Viskosität) [mPas] |
|---|---|---|---|---|---|---|---|---|---|---|
| 2 | 135 | 15 | Acrylsäure | 0,06 | Pentaerythrit-triallylether | 0,075 ABDV 1) | 70 | 140 | 10 | 2800 |
| 3 | 135 | 15 | Acrylsäure | 0,06 | Pentaerythrit-triallylether | 0,038 ABDV | 60 | 135 | 10 | 5400 |
| 4 | 135 | 15 | Acrylsäure | 0,075 | Pentaerythrit-triallylether | 0,038 ABDV | 60 | 130 | 10 | 1800 |
| 5 | 135 | 15 | Acrylsäure | 0,06 | Divinyldioxan | 0,038 ABDV | 70 | 140 | 11 | 5000 |
| 6 | 135 | 15 | Acrylsäure | 0,075 | Pentaerythrit-triallylether | 0,15 TB PEH 2) | 80 | 160 | 5 | 11600 |
| 7 | 135 | 15 | Acrylsäure | 0,075 | Pentaerythrit-triallylether | 0,15 AIBN 3) | 80 | 160 | 5 | 4800 |
| 8 | 135 | 12<br>3 | Acrylsäure<br>Methacrylsäure | 0,06 | Pentaerythrit-triallylether | 0,038 ABDV | 60 | 120 | 10 | 26400 |
| 9 | 135 | 12<br>3 | Acrylsäure<br>Methacrylsäure | 0,075 | Pentaerythrit-triallylether | 0,038 ABDV | 60 | 120 | 10 | 9600 |

Tabelle (Forts.)

| Beispiel | $CO_2$ [g] | Monomer (a) [g] | 0 ggf. (c) | Monomer (b) [g] | | Initiator [g] | Temp. [°C] | Druck [bar] | Zeit [h] | Verdickungswirkung (Viskosität) [mPas] |
|---|---|---|---|---|---|---|---|---|---|---|
| 10 | 135 | 12<br>3 | Acrylsäure<br>Methacrylsäure | 0,06 | Pentaerythrit-triallylether | 0,038 ABDV | 60 | 120 | 10 | 16200 |
| 11 | 135 | 15 | Methacrylsäure | 0,10 | Pentaerythrit-triallylether | 0,038 ABDV | 60 | 120 | 10 | 1800 |
| 12 | 120 | 30 | Acrylsäure | 0,12 | Pentaerythrit-triallylether | 0,3 ABDV | 70–110 | 145–200 | 10 | 3400 |
| 13 | 135 | 13,5<br>1,5 | Acrylsäure<br>Laurylacrylat | 0,075 | Pentaerythrit-triallylether | 0,075 ABDV | 70 | 140 | 10 | 9500 |
| 14 | 135 | 13,5<br>1,5 | Acrylsäure<br>Maleinsäure-anhydrid | 0,075 | Pentaerythrit-triallylether | 0,075 ABDV | 70 | 140 | 10 | 2300 |
| 15 | 135 | 9<br>3<br>3 | Acrylsäure<br>Methacrylsäure<br>Acrylamid | 0,06 | Pentaerythrit-triallylether | 0,038 ABDV | 60 | 120 | 10 | 8600 |
| 16 | 135 | 11,5<br>1,5<br>1,5<br>1,5 | Acrylsäure<br>Dimethyl-aminoethyl-acrylat<br>Methacrylamid<br>Acrylamido-methylpropyl sulfonsäure | 0,06 | Pentaerythrit-triallylether | 0,038 ABDV | 60 | 120 | 10 | 2300 |

1) ABDV = 2,2'-Azobis-(2,4-dimethylvaleronitril)
2) TB PEH = Tertiärbutylperethylhexanoat
3) AIBN = 2,2'-Azobissobutyronitril

**Patentansprüche**

1. Verfahren zur Herstellung von pulverförmigen vernetzten Copolymerisaten durch Polymerisieren von Monomerenmischungen aus
(a) 70 bis 99,999 Gew.% monoethylenisch ungesättigten Carbonsäuren, Amiden von monoethylenisch ungesättigten Carbonsäuren und/oder Estern aus monoethylenisch ungesättigten Carbonsäuren und Aminoalkoholen der Formel

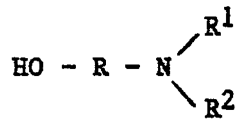

,

in der
R = $C_2$- bis $C_6$-Alkylen,
$R^1$, $R^2$ = H, $CH_3$, $C_2H_5$, $C_3H_7$ bedeutet,
(b) 0,001 bis 10 Gew.% eines mindestens zweifach ethylenisch ungesättigten Monomeren und
(c) 0 bis 20 Gew.% anderen monoethylenisch ungesättigten Monomeren
in einem inerten Verdünnungsmittel in Gegenwart von Radikale bildenden Initiatoren und Entfernen des Verdünnungsmittels, dadurch gekennzeichnet, daß man die Copolymerisation unter Druck in überkritischem Kohlendioxid als inertem Verdünnungsmittel unter Durchmischung bei Temperaturen bis 150°C und Drücken oberhalb von 73 bar durchführt und auf 100 Gew.-Teile der Monomeren 100 bis 150 Gew.-Kohlendioxid einsetzt.

2. Verfahren nach Anspruch 1, dadurch gekennzeichnet, daß man auf 100 Gew.-Teile der Monomeren 200 bis 900 Gew.-Teile Kohlendioxid einsetzt.

3. Verfahren nach den Ansprüchen 1 und 2, dadurch gekennzeichnet, daß man als Monomerenmischung
(a) monoethylenisch ungesättigte $C_3$- bis $C_5$-Carbonsäuren, Amide von ethylenisch ungesättigten $C_3$- bis $C_5$-Carbonsäuren und/oder Di-$C_1$- bis $C_3$-alkylamino-$C_2$- bis $C_6$-alkylacrylate oder -methacrylate in Form der Salze oder in quaternisierter Form,
(b) Divinyldioxan, Polyallyl- und Polyvinylether von Mono-, Di- und Oligosacchariden, N,N'-Methylen-bis-acrylamid, N,N'-Methylen-bis-methacrylamid, Di- und Triallylether von Trimethylolpropan und Glycerin, sowie Pentaerythrittriallylether und
(c) Acrylnitril, Methacrylnitril, Acrylsäure- und Methacrylsäureester von einwertigen $C_1$- bis $C_{18}$-Alkoholen, Hydroxy-$C_2$- bis $C_4$-alkylester der Acrylsäure und Methacrylsäure, Maleinsäureanhydrid, Vinylester und/oder Vinylphosphonsäure
einsetzt.

4. Verfahren nach den Ansprüchen 1 und 2, dadurch gekennzeichnet, daß man als Monomerenmischung
(a) 70 bis 99,95 Gew.% Acrylsäure, Methacrylsäure, Acrylamid und/oder Methacrylamid,
(b) 0,05 bis 5 Gew.% Divinyldioxan, Di-, Tri-, Tetra- und Pentaallylsaccharose oder deren Gemische, Pentaerythrittriallylether, N,N'-Methylen-Bisacrylamid und/oder
(c) 0 bis 20 Gew.% Acrylsäure- und Methacrylsäureester von einwertigen $C_1$- bis $C_{18}$-Alkoholen, Hydroxy-$C_2$- bis $C_4$-alkylester der Acrylsäure und Methacrylsäure, Maleinsäureanhydrid, Vinylacetat, Vinylpropionat und/oder Vinylphosphonsäure
einsetzt.

5. Verfahren nach den Ansprüchen 1 und 2, dadurch gekennzeichnet, daß man als Monomerenmischung
(a) Acrylsäure oder Methacrylsäure und
(b) Pentaerythrittriallylether oder N,N'-Methylen-bisacrylamid
einsetzt.

6. Verfahren nach den Ansprüchen 1 bis 5, dadurch gekennzeichnet, daß man als Radikale bildende Initiatoren tert.-Butylper-2-ethylhexanoat, 2,2'-Azobis(2,4-dimethylvaleronitril), 2,2'-Azobis (4-methoxi-2,4-dimethylvaleronitril), 2,2'-Azobisisobutyronitril und 2,2'-Azobis(2-amidinopropan)-dihydrochlorid einsetzt.

7. Verwendung der nach den Ansprüchen 1 bis 6 erhältlichen vernetzten Copolymerisate als Verdickungsmittel für wäßrige Systeme.

8. Verwendung der nach den Ansprüchen 1 bis 6 erhältlichen vernetzten Copolymerisate als Verdickungsmittel in kosmetischen und pharmazeutischen Zubereitungen.

## Claims

1. A process for the preparation of a pulverulent crosslinked copolymer, comprising polymerization of a monomer mixture consisting of

(a) from 70 to 99.999% by weight of monoethylenically unsaturated carboxylic acids, amides of such carboxylic acids and/or esters of such carboxylic acids and aminoalcohols of the formula

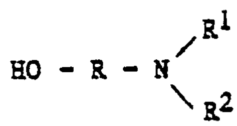

where R is $C_2$–$C_6$-alkylene and $R^1$ and $R^2$ are each H, $CH_3$, $C_2H_5$ or $C_3H_7$,
(b) from 0.001 to 10% by weight of a diethylenically or polyethylenically unsaturated monomer and
(c) from 0 to 20% by weight of other monoethylenically unsaturated monomers
in an inert diluent in the presence of a free radical initiator, and removal of the diluent, wherein the copolymerization is carried out under superatmospheric pressure in supercritical carbon dioxide as the inert diluent, with thorough mixing, at up to 150°C and under a pressure above 73 bar, and from 100 to 1,500 parts by weight of carbon dioxide are used per 100 parts by weight of the monomers.

2. A process as claimed in claim 1, wherein from 200 to 900 parts by weight of carbon dioxide are used per 100 parts by weight of the monomers.

3. A process as claimed in claims 1 and 2, wherein
(a) monoethylenically unsaturated $C_3$–$C_5$-carboxylic acids,
amides of ethylenically unsaturated $C_3$–$C_5$-carboxylic acids and/or di-$C_1$–$C_3$-alkylamino-$C_2$–$C_6$-alkyl acrylates or methacrylates in the form of the salts or in quaternized form,
(b) divinyldioxane, polyallyl and polyvinyl ethers of mono-, di- and oligosaccharides, N,N′-methylenebisacrylamide, N,N′-methylenebismethacrylamide, di- and triallyl ethers of trimethylolpropane and glycerol, and pentaerythritol triallyl ether, and
(c) acrylonitrile, methacrylonitrile, acrylates and methacrylates of monohydric $C_1$–$C_{18}$-alcohols, hydroxy-$C_2$–$C_4$-alkyl esters of acrylic acid and methacrylic acid, maleic anhydride, vinyl esters and/or vinylphosphonic acid are used as the monomer mixture.

4. A process as claimed in claims 1 and 2, wherein
(a) from 70 to 99.95% by weight of acrylic acid, methacrylic acid, acrylamide and/or methacrylamide,
(b) from 0.05 to 5% by weight of divinyldioxane, di-, tri-, tetra- and pentaallyl sucrose or mixtures of these, pentaerythritol triallyl ether or N,N′-methylenebisacrylamide and/or
(c) from 0 to 20% by weight of acrylates and methacrylates of monohydric $C_1$–$C_{18}$-alcohols, hydroxy-$C_2$–$C_4$-alkyl esters of acrylic acid and methacrylic acid, maleic anhydride, vinyl acetate, vinyl propionate and/or vinylphosphonic acid
are used as the monomer mixture.

5. A process as claimed in claims 1 and 2, wherein
(a) acrylic acid or methacrylic acid and
(b) pentaerythritol triallyl ether or N,N′-methylenebisacrylamide are used as the monomer mixture.

6. A process as claimed in any of claims 1 to 5, wherein tert.-butyl per-2-ethylhexanoate, 2,2′-azobis(2,4-dimethyl-valeronitrile), 2,2-azobis-(4-methoxy-2,4-dimethyl-valeronitrile), 2,2′-azobisisobutyronitrile or 2,2′-azobis-(2-amidinopropane) dihydrochloride is used as the free radical initiator.

7. Use of a crosslinked copolymer obtainable as claimed in any of claims 1 to 6 as a thickener for aqueous systems.

8. Use of a crosslinked copolymer obtainable as claimed in any of claims 1 to 6 as a thickener in cosmetic and pharmaceutical formulations.

## Revendications

1. Procédé de préparation de copolymères réticulés en poudre par polymérisation de mélanges de monomères composés de

(a) 70 à 99,999% en poids d'acides carboxyliques insaturés monoéthyléniquement, d'amides d'acides carboxyliques insaturés monoéthyléniquement et/ou d'esters d'acides carboxyliques insaturés monoéthyléniquement et d'amino-alccols de de formule

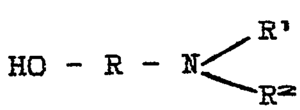

dans laquelle

R = alkylène en $C_2$ à $C_6$
$R^1$, $R^2$ = H, $CH_3$, $C_2H_5$, $C_3H_7$,
(b) 0,001 à 10% en poids d'un monomère insaturé éthyléniquement au moins deux fois et
(c) 0 à 20% en poids d'autres monomères insaturés monoéthyléniquement
dans un diluant inerte en présence d'initiateurs formant des radicaux, et par élimination du diluant, caractérisé en ce qu'on conduit la copolymérisation sous pression, dans l'anhydride carbonique à l'état surcritique servant de diluant inerte, avec mélange, à des températures allant jusqu'à 150°C et sous des pressions supérieures à 73 bars, et en ce qu'on utilise de 100 à 1500 parties en poids d'anhydride carbonique pour 100 parties en poids des monomères.

2. Procédé selon la revendication 1, caractérisé en ce qu'on utilise de 200 à 900 parties en poids d'anhydride carbonique pour 100 parties en poids des monomères.

3. Procédé selon la revendication 1 ou 2, caractérisé en ce qu'on utilise, comme mélange de monomères,
(a) des acides carboxyliques en $C_3$ à $C_5$ insaturés monoéthyléniquement, des amides d'acides carboxyliques en $C_3$ à $C_5$ insatures monoéthyléniquement et/ou des acrylates ou méthacrylates de di-($C_1$–$C_3$-alkyl)amino-$C_2$–$C_6$-alkyle sous forme des sels ou sous forme quaternisée,
(b) du divinyldioxanne, des polyallyl- et polyvinyléthers de mono-, di- et oligosaccharides, du N,N'-méthylène-bis-acrylamide, du N,N'-méthylène-bis-méthacrylamide, des di- et triallyléthers de triméthylolpropane et de glycérine, ainsi que des triallyléthers de pentaérythritol et
(c) de l'acrylonitrile, du méthacrylonitrile, des esters d'acide acrylique et méthacrylique et de monoalcools en $C_1$ à $C_{18}$, des esters hydroxyalkyliques en $C_2$–$C_4$ de l'acide acrylique et de l'acide méthacrylique, de l'anhydride maléique, un ester vinylique et/ou de l'acide vinylphosphonique.

4. Procédé selon la revendication 1 ou 2, caractérisé en ce qu'on utilise, comme mélange de monomères,
(a) de 70 à 99,95% en poids d'acide acrylique, d'acide méthacrylique, d'acrylamide et/ou de méthacrylamide,
(b) de 0,05 à 5% en poids de divinyldioxanne, de di-, tri-, tétra- et penta-allylsaccharose ou de leurs mélanges, de triallyléther de pentaérythritol, de N,N'-méthylène-bisacrylamide et/ou
(c) de 0 à 20% en poids d'esters d'acide acrylique et méthacrylique et de monoalcools en $C_1$ à $C_{18}$, d'esters hydroxyalkyliques en $C_2$–$C_4$ de l'acide acrylique et de l'acide méthacrylique, d'anhydride maléique, d'acétate de vinyle, de propionate de vinyle et/ou d'acide vinylphosphonique.

5. Procédé selon la revendication 1 ou 2, caractérisé en ce qu'on utilise, comme mélange de monomères,
(a) de l'acide acrylique ou de l'acide méthacrylique et
(b) du triallyléther de pentaérythritol ou du N,N'-méthylène-bis-acrylamide.

6. Procédé selon l'une quelconque des revendications 1 à 5, caractérisé en ce qu'on utilise, comme initiateurs formant des radicaux, du per-2-éthylhexanoate de butyle tertiaire, du 2,2'-azobis(2,4-diméthylvaléronitrile), du 2,2'-azobis(4-méthoxy-2,4-diméthylvaléronitrile), du 2,2'-azobisisobutyronitrile et du dichlorhydrate de 2,2'-azobis(2-amidinopropane).

7. Utilisation des copolymères réticulés obtenus selon l'une quelconque des revendications 1 à 6 comme épaississants pour des systèmes aqueux.

8. Utilisation des copolymères réticulés obtenus selon l'une quelconque des revendications 1 à 6 comme épaississants dans des préparations cosmétiques et pharmaceutiques.